# EUROPEAN PATENT SPECIFICATION

(11) **EP 4 559 446 B1**
(45) Date of publication and mention of the grant of the patent: **18.03.2026**
(21) Application number: 24213944.2
(22) Date of filing: 19.11.2024
(51) Int. Cl.: A61F 13/15, A61F 13/514, A61F 13/56, A61F 13/476, A61F 13/58

(54) **METHOD AND APPARATUS FOR MAKING AN ABSORBENT SANITARY ARTICLE**
VERFAHREN UND VORRICHTUNG ZUR HERSTELLUNG EINES SAUGFÄHIGEN HYGIENEARTIKELS
PROCÉDÉ ET APPAREIL DE FABRICATION D'UN ARTICLE HYGIÉNIQUE ABSORBANT

(30) Priority: 22.11.2023 IT 202300024807
(43) Date of publication of application: 28.05.2025
(73) Proprietor: GDM S.p.A., 40133 Bologna (IT)
(72) Inventor: ZAVALLONI, Alessandro, I-40133 Bologna (IT); TOSCANI, Federico, I-40133 Bologna (IT); ROSSI, Eros, I-40133 Bologna (IT)
(74) Representative: Porta & Consulenti Associati S.p.A.

(56) References cited:
- EP-A1- 0 589 437
- WO-A1-2013/018653
- US-A1- 2017 112 678
- US-A1- 2021 045 943

## Description

The present invention relates to a method and apparatus for making an absorbent sanitary article.

Absorbent sanitary items are for example female sanitary towels, nappies for babies, nappies for persons suffering from incontinence and the like. In the following description, reference will be made to the non-limiting example of female sanitary towels.

Typically, female sanitary towels comprise a top layer and a bottom layer, called "topsheet" and "backsheet" respectively in industry jargon, overlapping and joined together. An absorbent core is placed between the topsheet and backsheet.

The topsheet is made of a material permeable to body fluids and is configured to face the user's body, in contact with it, when the sanitary towel is applied to the undergarment.

The absorbent core has the function of absorbing the body fluids that pass through the topsheet and of retaining them without leaks even for several hours. The absorbent core comprises an absorbent material typically in granular or powder form, called SAP (acronym for "Super Absorbent Powder"), capable of absorbing liquids up to 300 times its own weight and up to 60 times its own volume.

The backsheet is made impermeable to bodily fluids to prevent leakage of the absorbed liquids from the absorbent core.

The female sanitary towel is configured to be applied to a user's undergarment by means of adhesive portions placed on the backsheet.

In a typical configuration, the female sanitary towel comprises a pair of wings arranged laterally to the absorbent core. First adhesive portions (also referred to in the following as "central adhesive portions") are located in a central region of the backsheet, overlaid on the absorbent core. Second adhesive portions (also referred to in the following as "peripheral adhesive portions") are placed on the backsheet at the wings, not overlapping the absorbent core.

The female sanitary towel is fixed inside the undergarment by the first adhesive portions. Subsequently, the wings are folded and turned over the outside of the undergarment and attached to it via the second adhesive portions.

To make an absorbent article, a multilayer web is formed by superimposing a backsheet web, in the form of a continuous web, a plurality of successively arranged absorbent cores, and a topsheet web, in the form of a continuous web.

The topsheet and backsheet webs are joined together by arranging the absorbent cores between them. Subsequently, the multilayer web is cut around each absorbent core to make a plurality of semi-finished absorbent articles. For each semi-finished absorbent article, the cut topsheet web defines the topsheet of the absorbent article, the cut backsheet web defines the backsheet of the absorbent article, and an absorbent core remains interposed between them.

Conventionally, the central and peripheral adhesive portions are applied to the backsheet web of the multilayer web formed as shown above, after the topsheet web and backsheet web have been joined with the absorbent cores arranged between them.

Each adhesive portion (central and/or peripheral) typically comprises a glue layer and a removable covering sheet overlaid on the glue layer. The adhesive portion is applied by attaching the glue layer to the backsheet web. When the covering sheet is removed by the user, the glue layer remains attached to the backsheet web and is exposed so that the absorbent article can be attached to the undergarment.

Typically, conventional synthetic-based glues are used for the glue layer. Such glues are solid at room temperature and are temporarily heated to a liquid state when they are to be applied.

In markets that are more sensitive to environmental issues, regulators and consumers prefer biodegradable products.

Therefore, biodegradable female sanitary towels have recently been developed that can be disposed of in toilet bowls and dissolved in water in the order of several days, weeks or months. In these products, the two topsheet and backsheet webs and the absorbent core are made of biodegradable materials, e.g. cellulose-based.

The Applicant has found that conventional synthetic-based glues are not biodegradable.

The Applicant has thought that, in order to make the aforementioned adhesive portions while maintaining the biodegradable properties of the product, glues of a biodegradable type could be used instead of conventional synthetic-based glues.

The Applicant noted that biodegradable glues currently in use are typically water-based and, at room temperature, are liquid and have particularly low viscosity, unlike conventional glues. Biodegradable glues generally also take longer to dry, even in the order of several days, and therefore remain liquid much longer than conventional glues after being applied. Biodegradable glues therefore generally have lower adhesion power than conventional glues during the processing steps following the application of the above-mentioned adhesive portions and at least until they harden.

The Applicant has found that it is possible to adequately attach the adhesive portions to the backsheet web made of biodegradable material by pressing the adhesive portions onto the backsheet web and exerting adequate pressure on them.

The Applicant verified, however, that compressing the adhesive portions directly on the multilayer web with such adequate pressure tends to crush and stiffen the absorbent cores, reducing comfort for the user.

The Applicant found that the stiffening of the absorbent core is further exacerbated when the multilayer web is also formed using low-viscosity biodegradable glues, since such glues tend, due to pressure, to penetrate the absorbent core, impregnating it.

The Applicant also found that the stiffening of the absorbent core is further exacerbated if the production speed is increased.

The Applicant has deduced that if the adhesive portions were applied to the backsheet web before forming the multilayer web, it would be possible to compress the adhesive portions against the backsheet web with even quite high pressure without causing the absorbent core to stiffen.

WO 2013/018653A1 discloses a prior art example of a method for producing an absorbent article.

The present invention therefore relates, in a first aspect thereof, to a method for making an absorbent sanitary article according to claim 1.

In a second aspect thereof, the present invention relates to an apparatus for making an absorbent sanitary article according to claim 6.

In the present invention, the adhesive portions are applied to the backsheet web before forming the multilayer web of the absorbent sanitary article. Thus, the absorbent cores of the multilayer web are not crushed during pressing of the adhesive portions onto the backsheet web. In this way, the adhesive portions can be pressed against the backsheet web with even high pressure, greatly improving the adhesion of the glue layer on it, without altering the characteristics of the absorbent core. The overall production speed can also be increased, without changing the characteristics of the absorbent core.

The apparatus of the second aspect of the present invention enables implementation of the method according to the first aspect of the present invention.

The present invention may have, in both aspects discussed above, at least one of the preferred features described below. Such features may thus be present individually or in combination with each other, unless expressly specified otherwise, both in the method of the first aspect of the present invention and in the apparatus of the second aspect of the present invention.

Preferably, said first pressing members are configured to press said adhesive portions against said backsheet web with said first force.

Preferably, said predetermined value is selected so that the adhesive portions are firmly adhered to the backsheet web.

Preferably, the predefined value of said first force is greater than 100 N.

In an embodiment, after pressing said adhesive portions against said backsheet web and before forming said multilayer web, said adhesive portions are pressed again against said backsheet web to firmly attach the adhesive portions to the backsheet web.

Preferably, pressing said adhesive portions against said backsheet web comprises passing said backsheet web and said adhesive portions between a pair of first pressing members.

Preferably, said pair of first pressing members comprises a pair of counter-rotating rollers.

Preferably, the pressing members of said first pair of pressing members are substantially in contact with each other.

Preferably, repeating pressing said adhesive portions against said backsheet web comprises passing said backsheet web and said adhesive portions between a further pair of first pressing members.

Preferably, said further pair of first pressing members comprises a further pair of counter-rotating rollers.

Preferably, the first pressing members of said further pair are substantially in contact with each other.

"Substantially in contact with each other" means that the first pressing members of the first pair of pressing members or the further pair of first pressing members are in contact with each other or spaced at most by the thickness of a single backsheet web and the adhesive portions applied thereto.

Preferably, the value of said second force is selected in such a way that it does not lead to appreciable stiffening of the absorbent cores.

Preferably, said second pressing members are configured to press said backsheet web towards said topsheet web with absorbent cores interposed between said backsheet web and said topsheet web.

Preferably, pressing said backsheet web against said topsheet web comprises passing said backsheet web, said topsheet web and said absorbent cores between a pair of second pressing members.

Preferably, the second pressing members of said pair of second pressing members are spaced out.

Preferably, the second pressing members of said pair of second pressing members are spaced more than 0.5 mm apart, preferably more than 1 mm, even more preferably more than 1.2 mm.

Preferably, after forming said multilayer web, the absorbent cores of said multilayer web are not compressed between pressing members that are substantially in contact with each other.

Preferably, said backsheet web comprises a central area extending astride and along a center axis of said backsheet web.

Preferably, said plurality of adhesive portions comprises a plurality of central adhesive portions.

Preferably, applying said plurality of adhesive portions comprises applying said plurality of central adhesive portions at said central area.

Preferably, said plurality of central adhesive portions are applied in such a manner that, after formation of the multilayer web, each central adhesive portion is overlaid on a respective absorbent core of said plurality of absorbent cores.

Preferably, said backsheet web comprises two peripheral areas arranged on opposite sides with respect to said central zone.

Preferably, said plurality of adhesive portions comprises a plurality of peripheral adhesive portions.

Preferably, applying said plurality of adhesive portions comprises applying said plurality of peripheral adhesive portions at said two peripheral areas.

Preferably, following the formation of said multilayer web, said two peripheral areas are cut to define a plurality of wings of absorbent sanitary articles.

Preferably, following the formation of said multilayer web, the multilayer web is cut to define a plurality of absorbent sanitary articles.

Preferably, each semi-finished absorbent sanitary article comprises a topsheet defined by a portion of said topsheet web, a backsheet defined by a portion of said backsheet web, and an absorbent core of said plurality of absorbent cores.

Preferably, cutting said multilayer web to define a plurality of semi-finished absorbent sanitary articles comprises cutting said two peripheral areas to define said plurality of wings of absorbent sanitary articles.

Preferably, each semi-finished absorbent sanitary article comprises a pair of wings protruding on opposite sides at the respective absorbent core.

Preferably, said wings comprise respective peripheral adhesive portions of said plurality of peripheral adhesive portions.

Preferably, cutting said two peripheral areas to define a plurality of wings includes cutting said two peripheral areas around respective peripheral adhesive portions previously applied.

Preferably, each wing is defined by a portion of the topsheet and a portion of the backsheet of the absorbent sanitary article. These portions are overlaid on top of each other, preferably directly in contact with each other.

Preferably, there is no absorbent core, or part of it, interposed between the portion of the topsheet and the portion of the backsheet defining the wing.

Preferably, said plurality of peripheral adhesive portions are applied in such a way that, after formation of the multilayer web, each peripheral adhesive portion is placed on a respective wing.

Preferably, said plurality of wings comprises a plurality of pairs of opposing wings.

Preferably, the wings of each pair of wings are folded towards each other.

Preferably, folding the wings of each pair of wings towards each other comprises folding said wings over said topsheet.

Preferably, an adhesive patch is applied to the peripheral adhesive portions of the folded wings of each pair of wings to join them together.

Preferably, said adhesive patch is configured to keep the wings of the respective pair of wings folded.

Preferably, said adhesive patch is applied to the peripheral adhesive portions of said wings so as to adhere to said covering sheets.

Preferably, the covering sheets of the peripheral adhesive portions of each pair of wings are removable together with the respective adhesive patch by removing the adhesive patch from the absorbent sanitary article in one motion.

Preferably, the covering sheets of the peripheral adhesive portions of each pair of wings are removable together with the respective adhesive patch so that the respective adhesive layers remain on the respective wings.

Preferably, following the application of said plurality of adhesive portions and prior to the formation of said multilayer web, a layer of polyvinyl alcohol is applied to said backsheet web on the side opposite said plurality of adhesive portions.

Preferably, the layer of polyvinyl alcohol is configured to waterproof the backsheet during use of the absorbent sanitary article.

Preferably, the layer of polyvinyl alcohol is configured to dissolve in water after a predetermined period of time.

Preferably, said plurality of adhesive portions comprises a plurality of central adhesive portions.

Preferably, said adhesive portions applicator is configured to apply said plurality of central adhesive portions at a central area of said backsheet web extending astride and along a center axis of said backsheet web.

Preferably, said plurality of adhesive portions comprises a plurality of peripheral adhesive portions.

Preferably, said adhesive portions applicator is configured to apply said plurality of peripheral adhesive portions at two peripheral areas of said backsheet web arranged on opposite sides with respect to a central zone of said backsheet web extending astride and along a center axis of said backsheet web.

Preferably, said apparatus further comprises an outlet path.

Preferably, said outlet path is arranged downstream of said forming unit along said advancement direction.

Preferably, said output path is configured to receive said multilayer web from said forming unit.

Preferably, said apparatus further comprises a cutting member.

Preferably, said cutting member is arranged along said outlet path.

Preferably, said cutting member is configured to cut said peripheral areas to define a plurality of wings of absorbent sanitary articles comprising respective peripheral adhesive portions of said plurality of peripheral adhesive portions.

Preferably, said plurality of wings comprises a plurality of pairs of opposing wings.

Preferably, said apparatus further comprises a folding member.

Preferably, said folding member is placed along said outlet path.

Preferably, said folding member is configured to fold the wings of each pair of wings towards each other.

Preferably, said apparatus further comprises an applicator of adhesive patches.

Preferably, said an applicator of adhesive patches is placed along said outlet path.

Preferably, said applicator of adhesive patches is configured to apply an adhesive patch to the peripheral adhesive portions of the folded wings of each pair of wings to join said folded wings to each other.

Preferably, said apparatus further includes an applicator of polyvinyl alcohol layer.

Preferably, said applicator of polyvinyl alcohol layer is placed along said feeding path between said adhesive portions applicator and said forming unit.

Preferably, said applicator of polyvinyl alcohol layer is configured to apply a layer of polyvinyl alcohol to said backsheet web on the opposite side to said adhesive portions.

Preferably, said topsheet web and/or said backsheet web and/or said absorbent core are made of biodegradable material. In this way, the absorbent sanitary article multilayer web is, at least partially, biodegradable.

Preferably, said glue is biodegradable glue. In this way, a fully biodegradable absorbent sanitary article can be produced.

Further features and advantages of the present invention will become clearer from the following detailed description of a preferred embodiment thereof, with reference to the appended drawings and provided by way of indicative and non-limiting example, in which:
- Figure 1 is a schematic view of a part of an apparatus for making an absorbent sanitary article according to the present invention;
- Figure 2 is a schematic view of another part of the apparatus of the present invention;
- Figure 2A is a schematic plan view of a portion of the backsheet web used in the apparatus of the present invention;
- Figure 3 is an exploded perspective view of an absorbent sanitary article made using the apparatus of the present invention;
- Figure 4 is a schematic sectional view of the absorbent sanitary article in Figure 3.

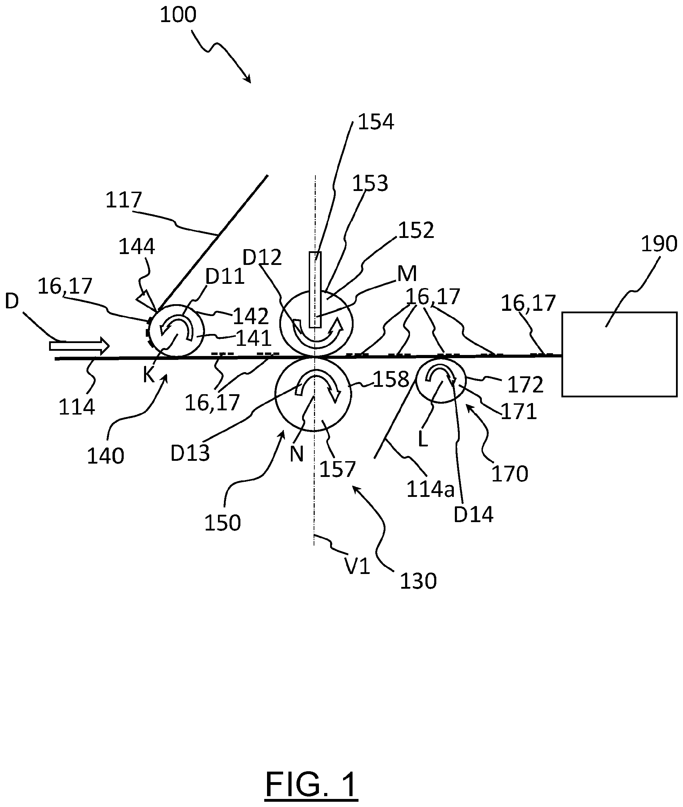

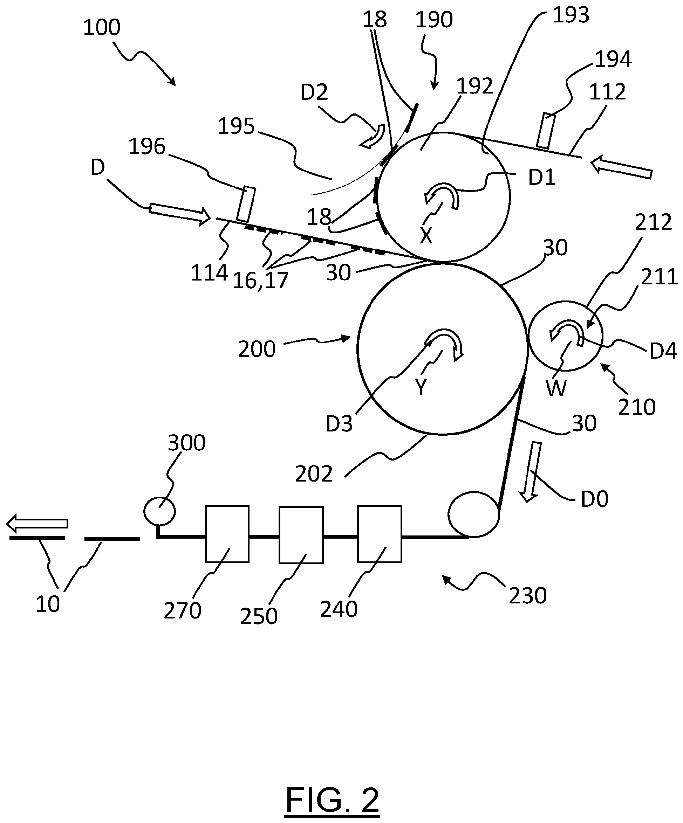

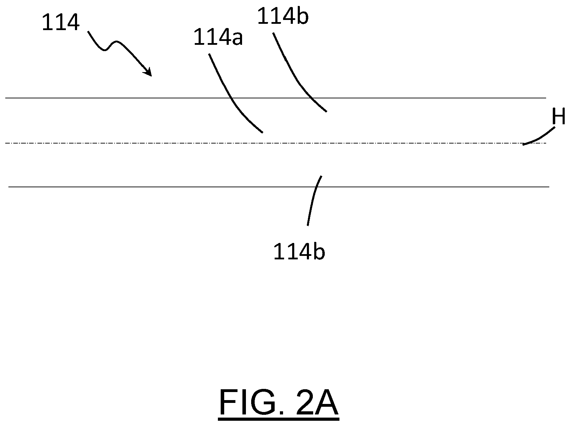

In Figures 1 and 2, the numerical reference 100 is used to indicate an apparatus for making an absorbent sanitary article in accordance with the present invention.

Non-limiting examples of absorbent sanitary articles made by the apparatus 100 are female sanitary towels, nappies for babies, nappies for persons suffering from incontinence and the like.

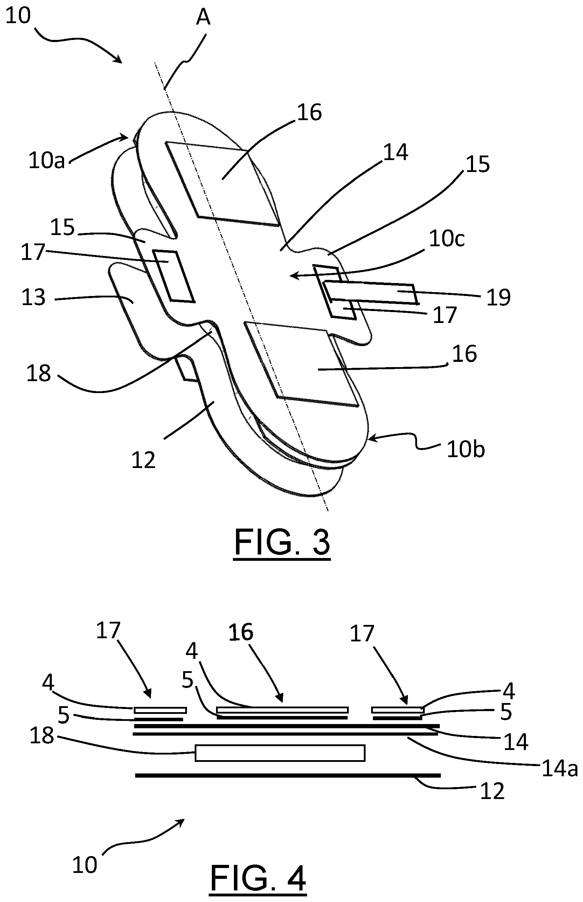

Without losing generality, explicit reference will be made below to a specific example of an absorbent sanitary article, such as the one illustrated in Figures 3 and 4 and indicated by numerical reference 10. It is specifically a feminine sanitary towel. When described, however, it can be similarly applied to different types of absorbent sanitary articles.

The article 10 has an elongated shape along a longitudinal axis A. With respect to the longitudinal axis A, the article 10 has a front part 10a, a rear part 10b and a central part 10c arranged between the front part 10a and the rear part 10b. The central part 10c has a sidecut with respect to the front part 10a and the rear part 10b.

The article 10 comprises a topsheet 12, also known as the "topsheet", and a backsheet 14, also known as the "backsheet", overlaid. The topsheet 12 and the backsheet 14 define, respectively, an inner face and an outer face of the article 10. The topsheet 12 and the backsheet 14 extend longitudinally from the front part 10a to the rear part 10b. The topsheet 12 and the backsheet 14 have the same shape and extension in plan as the shape and extension in plan of the article 10.

The topsheet 12 is made of a biodegradable material permeable to body fluids, preferably cellulose fibres.

The backsheet 14 is made of a biodegradable material, preferably cellulose fibres.

A waterproofing layer 14a is applied to the backsheet 14 on the side facing the topsheet 12 for the containment of liquids (Figure 4). The waterproofing layer 14a is configured to contain liquids for a predetermined period of time, e.g. several hours, days or weeks, and to dissolve in water if immersed for longer than the predetermined period of time. The waterproofing layer 14a is made of a water-soluble material, e.g. polyvinyl alcohol.

Two fastening wings 13, 15 protrude from opposite sides of the article 10 transverse to the longitudinal axis A. The fixing wings 13, 15 protrude at the central part 10c, in particular at its sidecut. The fastening wings 13, 15 are formed from respective portions of the topsheet 12 and backsheet 14. These portions are overlaid to each other and directly in contact with each other.

The article 10 can be applied to an undergarment by means of adhesive portions 16, 17 placed on the backsheet 14. The latter therefore faces the undergarment while the topsheet 12 faces away from the undergarment, towards the user's body.

Each adhesive portion 16, 17 comprises a removable covering sheet 4 and a glue layer 5 placed between the covering sheet 4 and the backsheet 14. The glue layer 5 holds the covering sheet 4 on the backsheet 14. The glue layer 5 comprises a water-based biodegradable glue. The covering sheet 4 is removable to expose the glue layer 5 that remains applied to the backsheet 14 and thus to allow the application of the article 10 to the undergarment.

The adhesive portions 16, 17 comprise central adhesive portions 16 placed at the longitudinal axis A and configured to be applied to the inside of the undergarment. In the embodiment illustrated in Figure 3, there are two central adhesive portions 16. One central adhesive portion 16 is placed at the front part 10a and the other central adhesive portion 16 is placed at the rear part 10b.

The adhesive portions 16, 17 further comprise, in an embodiment such as that illustrated in Figures 3 and 4, peripheral adhesive portions 17.

Such peripheral adhesive portions 17 are placed on the wings 13, 15, spaced with respect to the longitudinal axis A, and are configured to be applied to the outside of the undergarment by folding and turning back the two wings 13, 15. In the embodiment illustrated in Figures 3 and 4, there are two peripheral adhesive portions 17, one for each wing 13, 15.

An absorbent core 18 is arranged between the topsheet 12 and the backsheet 14. Such an absorbent core 18 has the function of absorbing the body fluids that pass through the topsheet 12 and of retaining them without leaks even for several hours. The absorbent core 18 is made of absorbent material comprising a fibre matrix, e.g. cellulose, and an absorbent powder (SAP) dispersed in the fibre matrix, e.g. carboxymethyl cellulose (CMC). Thus, the absorbent core 18 is biodegradable. The absorbent core 18 extends longitudinally from the front part 10a to the rear part 10b and is substantially oval in shape, with a sidecut at the central part 10c.

Figures 1 and 2 show parts of the apparatus 100 that enables the manufacture of the absorbent sanitary article 10.

The apparatus 100 comprises a forming unit 190 configured to form a multilayer web 30 of the absorbent sanitary article 10. The forming unit 190 is schematically illustrated in Figure 1 and is described in detail below with reference to Figure 2.

The multilayer web 30 comprises a topsheet web 112, a backsheet web 114 and a plurality of absorbent cores 18 interposed successively between the topsheet web 112 and the backsheet web 114.

Referring to Figure 2, the forming unit 190 comprises a forming drum 192 rotatable about the axis of rotation X. The forming drum 192 comprises an outer surface 193, preferably suctioned.

The forming drum 192 is configured to rotate in a direction D1 (which is anti-clockwise in Figure 2).

The apparatus 100 is configured to feed the topsheet web 112 onto the forming drum 192, in particular directly onto the outer surface 193. A first glue dispenser 194 is configured to dispense liquid glue, preferably water-based, onto the topsheet web 112 before the topsheet web 112 is supplied onto the forming drum 192, so as to impregnate it with glue. This glue is preferably biodegradable glue.

As shown in Figure 2, the forming unit 190 comprises a transport drum 195 (only partially shown) for transporting absorbent cores 18. The transport drum 195 is configured to hold a plurality of absorbent cores 18, preferably by suction, and to advance them in a direction D2 (which is clockwise in Figure 2), opposite the direction D1 of the forming drum 192, by rotating about its axis of rotation (not illustrated).

The transport drum 195 is substantially tangential to the forming drum 192 and is configured to deposit the absorbent cores 18 on the forming drum 192 by superimposing them with the topsheet web 112.

The apparatus 100 comprises a feeding path 130 (Figure 1) configured to feed the backsheet web 114 to the forming unit 190 by moving it along a feed direction D, shown in Figures 1 and 2. In particular, the feeding path 130 is configured to feed the backsheet web 114 onto the forming drum 192, superimposing it with the topsheet web 112 and the absorbent cores 18 overlaid on it. In this way, the absorbent cores 18 are interposed between the topsheet web 112 and the backsheet web 114 and the multilayer web 30 is formed.

The backsheet web 114 is moved along the feeding path 130, e.g. from a reel (not shown) loaded onto the apparatus 100 and progressively unwound. A plurality of diverter rollers configured to guide the backsheet web 114 to the forming unit 190 are provided along the feeding path 130.

As illustrated in Figure 2A, the backsheet web 114, when stretched along the feeding path 130, comprises a central zone 114a extending astride and along a center axis H of the backsheet web 114 and two peripheral areas 114b arranged on opposite sides with respect to the central area 114a.

As illustrated in Figure 1, an adhesive portions applicator 140 is arranged along the feeding path 130, configured to apply adhesive portions 16, 17 on the backsheet web 114 upstream of the forming unit 190. The adhesive portions 16, 17 are shaped as described above and are applied to one side of the backsheet web 114 which is opposite the side where, in the forming unit 190, the absorbent cores 18 are located.

The adhesive portions applicator 140 is configured to apply a plurality of central adhesive portions 16 at the central area 114a of the backsheet web 114 and, if provided (as in the example illustrated in Figures 3 and 4), also a plurality of peripheral adhesive portions 17 at the two peripheral areas 114b of the backsheet web 114.

As illustrated in Figure 1, the adhesive portions applicator 140 comprises a roller 141 rotatable about an axis of rotation K. The roller 141 comprises an outer surface 142, preferably suctioned. The roller 141 is configured to rotate about the axis of rotation K in a direction D11 (which is anticlockwise in Figure 1).

The central and peripheral adhesive portions 16, 17 are made by cutting into pieces an adhesive web 117 supplied to the roller 141 (or alternatively several adhesive webs, not shown), by means of a cutter 144 acting at the roller 141.

A pair of first pressing members 150 configured to press the central and peripheral adhesive portions 16, 17 against the backsheet web 114 is arranged along the feeding path 130, downstream of the adhesive portions applicator 140. The first pair of pressing members 150 exercises a force with a predetermined value, greater than 100 N.

Again with reference to Figure 1, the pair of first pressing members 150 comprises a compression roller 152 opposing an abutment roller 157.

The compression roller 152 rotates about the axis of rotation M in a direction D12 (which is anticlockwise in Figure 1). A preferably smooth compression surface 153 is defined on a cylindrical outer surface of the compression roller 152. In the remainder of the present description, a surface is understood to be "smooth" if it is a uniform surface free from recesses, protrusions, knurls, holes, roughness and other obvious unevenness, and with a surface roughness less than 100 µm, preferably less than 10 µm.

The abutment roller 157 rotates about the axis of rotation N, parallel to the axis of rotation M, in a direction D13 (which is clockwise in figure 1), opposite the direction D12 of the compression roller 152. A preferably smooth abutment surface 158 is defined on a cylindrical outer surface of the abutment roller 157.

In the non-limiting example shown in Figure 1, the axes of rotation M and N lie on a plane of track V1 which is essentially vertical.

The abutment roller 157 is located substantially tangential to the compression roller 152. The axes of rotation M and N lie in a plane that is radial for both the abutment roller 157 and the compression roller 152.

In the non-limiting example shown in Figure 1, the compression roller 152 and the abutment roller 157 have essentially the same outer diameter.

The compression roller 152 is pressed against the abutment roller 157 by a preloaded elastic element 154 so that the compression surface 153 is pressed against the abutment surface 158. The preloaded elastic element 154 comprises e.g. elastic springs or a pneumatic or hydraulic cylinder.

In an alternative, non-illustrated embodiment of the present invention, the pre-loaded elastic element 154 may be omitted. In such a case, preferably fine adjustment of the position of the compression roller 152 relative to the abutment roller 157 is provided, so that the compression surface 153 is substantially in contact with the abutment surface 158. The Applicant has verified that the smaller the distance between the compression roller 152 and the abutment roller 157, the greater the compression exerted by said rollers 152, 157 and the better the adhesion obtained between the adhesive portions 16, 17 and the backsheet web 114. By allowing the distance between the compression roller 152 and the abutment roller 157 to be adjusted, it is possible to adjust the degree of compression by balancing the opposing requirements of reducing glue leakage and improving adhesion.

In a further alternative non-illustrated embodiment of the present invention, there is provided a further pair of first pressing members 150 comprising a further compression roller opposed to a further abutment roller, entirely analogous to the compression roller 152 and abutment roller 157 described above. These further rollers are placed downstream of the compression roller 152 and the abutment roller 157 along the feeding path 130 and enable the adhesive portions 16, 17 to be further pressed against the backsheet web 114.

An applicator of polyvinyl alcohol layer 170 is arranged along the feeding path 130, downstream of the first pair of pressing members 150 and upstream of the forming unit 190. Such an applicator 170 is configured to apply a layer of polyvinyl alcohol 114a to the backsheet web 114 opposite the adhesive portions 16, 17. The layer of polyvinyl alcohol 114a implements the waterproofing layer 14a of articles 10 being produced.

As illustrated in Figure 1, the applicator of polyvinyl alcohol layer 170 comprises a roller 171 rotatable about an axis of rotation K. The roller 171 comprises an outer surface 172, preferably suctioned. The roller 171 is configured to rotate about the axis of rotation L in a direction D14 (which is clockwise in Figure 1).

As illustrated in Figure 2, a second glue dispenser 196 is configured to dispense liquid glue, preferably water-based, onto the backsheet web 114 before it is supplied onto the forming drum 192, in order to impregnate it with glue. This glue is preferably biodegradable glue. In Figure 2, the backsheet web 114 is illustrated upside down with respect to the backsheet web 114 in Figure 1.

In an alternative, non-illustrated embodiment of the present invention, only one of the first glue dispenser 194 and the second glue dispenser 196 could be provided so as to impregnate only one of the topsheet web 112 and the backsheet web 114.

The forming unit 190 further comprises second pressing members 210 configured to compress the multilayer web 30 downstream of the forming drum 192.

The second pressing members 210 comprise a support drum 200 positioned adjacent to the forming drum 192 and substantially tangent to it. The support drum 200 comprises, on a cylindrical outer surface thereof, a bearing surface 202, which is preferably smooth.

The support drum 200 is rotatable about an axis of rotation Y. The axis of rotation Y is parallel to the axis of rotation X. The support drum 200 is configured to rotate in a direction D3 (which is clockwise in Figure 2), opposite to the direction D2.

The bearing surface 202 has a width greater than or equal to the width of the multilayer web 30. The width of the bearing surface 202 is measured in the direction parallel to the axis of rotation Y of the support drum 200. The width of the multilayer web 30 is measured in a direction orthogonal to a main longitudinal direction of the multilayer web 30 and parallel to the axis of rotation Y of the support drum 200.

In the example shown in Figure 2, the forming drum 192 is placed above the support drum 200. The diameter of the forming drum 192 is smaller than the diameter of the support drum 200.

The forming drum 192 is configured to feed the multilayer web 30 onto the support drum 200. In particular, the multilayer web 30 is advanced in the direction D1 on the outer surface 193 of the forming drum 192 and passed between the forming drum 192 and the support drum 200. When the multilayer web 30 arrives between the forming drum 192 and the support drum 200, the multilayer web 30 is left by the forming drum 192, e.g. by ceasing the suction action towards the outer surface 193, taken over by the support drum 200 and advanced onto the bearing surface 202 in the direction D3.

In the example illustrated in Figure 2, the second pressing members 210 comprise a compression roller 211 facing the support drum 200 and configured to press the multilayer web 30 against the bearing surface 202. The compression roller 211 has a cylindrical outer surface 212 which is preferably smooth.

The compression roller 211 is rotatably mounted on a respective support body (not shown), preferably in idle mode. The compression roller 211 is rotatable about an axis of rotation W parallel to the axis of rotation Y of the support drum 200. The compression roller 211 is configured to rotate about the axis of rotation W in a direction D4 (which is anticlockwise in Figure 2) opposite to the direction D3 of the support drum 200.

Preferably, the support body is connected to respective adjustment members (not shown) configured to allow adjustment of the position of the compression roller 211 with respect to the support drum 200. The adjustment members can comprise, for example, threaded parts such as bolts and nuts. The adjustment members are configured to allow the position of the compression roller 211 to be adjusted in a direction orthogonal to the bearing surface 202. This direction is radial with respect to the axis of rotation Y of the support drum 200.

The compression roller 211 is placed at a respective distance from the bearing surface 202. This distance defines a gap between the support drum 200 and the compression roller 211 through which the multilayer web 30 is passed. This distance can be adjusted by means of the adjustment members.

The multilayer web 30 is advanced on the bearing surface 202, in particular by rotation of the support drum 200, and passed between the bearing surface 202 and the compression roller 211, so as to be compressed between the two. The compression of the multilayer web 30 improves the adhesion between the individual glue-impregnated layers of the multilayer web 30.

The distance between the compression roller 211 and the bearing surface 202, meant as the height of the gap through which the multilayer web 30 is passed, is greater than 0.5 mm, preferably greater than 1 mm, even more preferably greater than 1.2 mm, so as not to excessively compress the absorbent cores 18.

The second pressing members 210 presses the backsheet web 114 towards the topsheet web 112 by exerting a force having a value less than the predetermined value exerted by the first pair of pressing members 150, so as not to impair the softness characteristics of the absorbent cores 18.

In an alternative, non-illustrated embodiment of the present invention, the second pressing members 210 comprise a further compression roller, analogous to the compression roller 211, which is placed downstream of the compression roller 211 with respect to a feed direction D0 of the multilayer web 30 on the bearing surface 202. This further compression roller is configured to press the multilayer web 30 against the bearing surface 202 one more time.

The distance between the further compression roller and the bearing surface 202, understood as the height of the gap through which the multilayer web 30 is passed, is less than the distance between the compression roller 211 and the bearing surface 202 and is greater than 0.5 mm, preferably greater than 1 mm, even more preferably greater than 1.2 mm, so as not to excessively compress the absorbent cores 18.

The apparatus 100 is configured to allow the multilayer web 30 to leave the bearing surface 202 downstream of the compression roller 211 and be moved along an outlet path 230 (Figure 2) for further processing.

The outlet path 230 is then arranged downstream of the forming unit 190 along the feed direction D0 of the multilayer web 30. In the outlet path 230 the multilayer web 30 can advance via conveyor belts (not shown).

Along the outlet path 230 a cutting member 240 is arranged configured to cut the multilayer web 30 at its opposing longitudinal peripheral areas to define, in succession, a plurality of pairs of opposing wings 13, 15 of the absorbent sanitary articles 10 being produced, wherein a respective pair of peripheral adhesive portions 17 is applied to each pair of wings 15. The longitudinal peripheral areas of the multilayer web 30 correspond to the peripheral areas 114b of the backsheet web 114. The wings 13, 15 are formed by cutting both the topsheet web 112 and the topsheet web 114 overlaid thereon.

Along the outlet path 230, downstream of the cutting member 240, a folding member 250 is arranged configured to fold the wings 13, 15 of each pair of wings 13, 15 towards each other by folding them over the topsheet web 114.

An adhesive patch applicator 270 configured to apply an adhesive patch 19 to both peripheral adhesive portions 17 of a pair of wings 13, 15 to join the folded wings 13, 15 together and prevent them from unfolding is arranged along the outlet path 230, downstream of the folding member 250.

In a final processing step, the multilayer web 30 is finally cut by a subdivision member 300, itself conventional, into discrete elements each corresponding to a single absorbent article 10 of the type described above.

Further processing stations can possibly be interposed between the support drum 200 and the subdivision member 300.

Obviously, a person skilled in the art, in order to satisfy specific and contingent needs, can make numerous modifications and variations to the invention described above while remaining within the scope of protection defined by the following claims.

## Claims

1. Method for making an absorbent sanitary article (10), comprising:
applying a plurality of adhesive portions (16, 17) on a backsheet web (114), each adhesive portion (16, 17) comprising a removable covering sheet (4) and a glue layer (5) overlaid on said covering sheet (4);
subsequently, forming an absorbent sanitary article multilayer web (30) comprising said backsheet web (114), a topsheet web (112), a plurality of absorbent cores (18) arranged between said topsheet web (112) and said backsheet web (114), and said plurality of adhesive portions (16, 17) applied to said backsheet web (114), wherein said adhesive portions (16, 17) are arranged on said backsheet web (114) on an opposite side with respect to said plurality of absorbent cores (18);
prior to forming said multilayer web (30), pressing said adhesive portions (16, 17) against said backsheet web (114) with a first force having a predetermined value; wherein forming said multilayer web (30) comprises pressing said backsheet web (114) against said topsheet web (112) with a second force having a value less than said predetermined value.

2. Method according to claim 1, wherein:
said backsheet web (114) comprises a central area (114a) extending astride and along a center axis (H) of said backsheet web (114);
applying said plurality of adhesive portions (16, 17) comprises applying a plurality of central adhesive portions (16) at said central area (114a).

3. Method according to any one of the previous claims, wherein:
said backsheet web (114) comprises a central area (114a) extending astride and along a center axis (H) of said backsheet web (114) and two peripheral areas (114b) arranged on opposite sides with respect to said central area (114a);
applying said plurality of adhesive portions (16, 17) comprises applying a plurality of peripheral adhesive portions (17) at said two peripheral areas (114b);
said method further comprising, subsequent to forming said multilayer web (30), cutting said two peripheral areas (114b) to define a plurality of wings (13, 15) of absorbent sanitary articles (10) comprising respective peripheral adhesive portions (17) of said plurality of peripheral adhesive portions (17).

4. Method according to claim 3, wherein said plurality of wings (13, 15) comprises a plurality of pairs of opposing wings (13, 15), said method comprising:
folding the wings (13, 15) of each pair of wings (13, 15) towards each other;
applying an adhesive patch (19) to the peripheral adhesive portions (17) of the folded wings (13, 15) of each pair of wings (13, 15) to join said folded wings (13, 15) together.

5. A method according to any one of the previous claims, comprising, subsequent to applying said plurality of adhesive portions (16, 17) and prior to forming said multilayer web (30), applying a layer of polyvinyl alcohol (14a) to said backsheet web (114) on the opposite side to said plurality of adhesive portions (16, 17).

6. Apparatus (100) for making an absorbent sanitary article (10), comprising:
a forming unit (190) configured to form a multilayer web (30) of absorbent sanitary article (10) comprising a backsheet web (114), a topsheet web (112) and a plurality of absorbent cores (18) arranged between said topsheet web (112) and said backsheet web (114);
a feeding path (130) arranged upstream of said forming unit (190) along an advancement direction of said backsheet web (114) and configured to feed said backsheet web (114) to said forming unit (190);
an adhesive portions applicator (140) arranged along said feeding path (130) and configured to apply a plurality of adhesive portions (16, 17) on said backsheet web (114), each adhesive portion (16, 17) comprising a removable covering sheet (4) and a glue layer (5) overlaid on said covering sheet (4);
first pressing members (150) arranged along said feeding path (130) and configured to press said adhesive portions (16, 17) against said backsheet web (114) with a first force having a predetermined value;
wherein said forming unit (190) comprises second pressing members (210) configured to press said backsheet web (114) towards said topsheet web (112) with a second force having a value less than said predetermined value.

7. Apparatus (100) according to claim 6, wherein
said plurality of adhesive portions (16, 17) comprises a plurality of central adhesive portions (16); and
said adhesive portions applicator (140) is configured to apply said plurality of central adhesive portions (16) at a central area (114a) of said backsheet web (114) extending astride and along a center axis (H) of said backsheet web (114).

8. Apparatus (100) according to claim 6 or 7, wherein:
said plurality of adhesive portions (16, 17) comprises a plurality of peripheral adhesive portions (17);
said adhesive portions applicator (140) is configured to apply said plurality of peripheral adhesive portions (17) at two peripheral areas (114b) of said backsheet web (114) arranged on opposite sides with respect to a central area (114a) of said backsheet web (114) extending astride and along a center axis (H) of said backsheet web (114);
said apparatus (100) further comprising:
an outlet path (230) arranged downstream of said forming unit (190) along said advancement direction and configured to receive said multilayer web (30) from said forming unit (190);
a cutting member (240) arranged along said outlet path (230) and configured to cut said peripheral areas (114b) to define a plurality of wings (13, 15) of absorbent sanitary articles (10) comprising respective peripheral adhesive portions (17) of said plurality of peripheral adhesive portions (17).

9. Apparatus (100) according to claim 8, wherein said plurality of wings (13, 15) comprises a plurality of pairs of opposing wings (13, 15), said apparatus (100) further comprising:
a folding member (250) placed along said outlet path (230) and configured to fold the wings (13, 15) of each pair of wings (13, 15) towards each other;
an applicator of adhesive patches (270) placed along said outlet path (230) and configured to apply an adhesive patch (19) to the peripheral adhesive portions (17) of the folded wings (13, 15) of each pair of wings (13, 15) to join said folded wings (13, 15) to each other.

10. Apparatus (100) according to any one of claims 6 to 9, comprising an applicator of polyvinyl alcohol layer (170) placed along said feeding path (130) between said adhesive portions applicator (140) and said forming unit (190) and configured to apply a layer of polyvinyl alcohol (14a) on said backsheet web (114) on the opposite side to said adhesive portions (16, 17).

## Patentansprüche

1. Verfahren zur Herstellung eines absorbierenden Hygieneartikels (10), umfassend:
Aufbringen einer Vielzahl von Klebeabschnitten (16, 17) auf eine Rückseitenbahn (114), wobei jeder Klebeabschnitt (16, 17) eine abnehmbare Deckschicht (4) und eine auf diese Deckschicht (4) aufgebrachte Klebeschicht (5) umfasst;
anschließendes Bilden einer mehrschichtigen Bahn (30) für einen absorbierenden Hygieneartikel, die die Rückseitenbahn (114), eine Oberseitenbahn (112), eine Vielzahl absorbierender Kerne (18), die zwischen der Oberschichtbahn (112) und der Unterschichtbahn (114) angeordnet sind, und eine Vielzahl von Klebeabschnitten (16, 17), die auf die Rückseitenbahn (114) aufgebracht sind,
wobei die Klebeabschnitte (16, 17) auf der Rückseitenbahn (114) auf einer gegenüberliegenden Seite im Hinblick auf die Vielzahl der absorbierenden Kerne (18) angeordnet sind;
vor dem Bilden der mehrschichtigen Bahn (30) das Andrücken der Klebeabschnitte (16, 17) an die Rückseitenbahn (114) mit einer ersten Kraft, die einen vorbestimmten Wert aufweist; wobei das Bilden der mehrschichtigen Bahn (30) das Andrücken der Rückseitenbahn (114) an die Oberseitenbahn (112) mit einer zweiten Kraft umfasst, die unterhalb des vorbestimmten Werts liegt.

2. Verfahren nach Anspruch 1, wobei:
die Rückseitenbahn (114) einen mittleren Bereich (114a) aufweist, der sich rittlings und entlang einer Mittelachse (H) der Rückseitenbahn (114) erstreckt;
das Aufbringen der Vielzahl von Klebeabschnitten (16, 17) das Aufbringen mehrerer mittlerer Klebeabschnitte (16) in dem mittleren Bereich (114a) umfasst.

3. Verfahren nach einem der vorhergehenden Ansprüche, wobei:
die Rückseitenbahn (114) einen mittleren Bereich (114a), der sich über eine Mittelachse (H) der Rückseitenbahn (114) erstreckt, und zwei periphere Bereiche (114b) umfasst, die auf gegenüberliegenden Seiten im Hinblick auf den mittleren Bereich (114a) angeordnet sind;
das Aufbringen der Vielzahl von Klebeabschnitten (16, 17) das Aufbringen einer Vielzahl von peripheren Klebeabschnitten (17) an den beiden peripheren Bereichen (114b) umfasst;
wobei das Verfahren nach dem Bilden der mehrschichtigen Bahn (30) ferner das Schneiden der beiden peripheren Bereiche (114b) umfasst, um eine Vielzahl von Flügeln (13, 15) von absorbierenden Hygieneartikeln (10) zu definieren, die jeweilige periphere Klebeabschnitte (17) der Vielzahl von peripheren Klebeabschnitten (17) umfassen.

4. Verfahren nach Anspruch 3, wobei die Vielzahl von Flügeln (13, 15) eine Vielzahl von Paaren gegenüberliegender Flügel (13, 15) umfasst, wobei das Verfahren umfasst:
Falten der Flügel (13, 15) eines jeden Flügelpaares (13, 15) zueinander;
Aufbringen eines Klebepflasters (19) auf die peripheren Klebebereiche (17) der gefalteten Flügel (13, 15) jedes Flügelpaares (13, 15), um die gefalteten Flügel (13, 15) miteinander zu verbinden.

5. Verfahren nach einem der vorhergehenden Ansprüche, umfassend, nach dem Aufbringen der Vielzahl von Klebeabschnitten (16, 17) und vor dem Bilden der mehrschichtigen Bahn (30), das Aufbringen einer Schicht aus Polyvinylalkohol (14a) auf die Rückseitenbahn (114) auf der der Vielzahl von Klebeabschnitten (16, 17) gegenüberliegenden Seite.

6. Vorrichtung (100) zur Herstellung eines absorbierenden Hygieneartikels (10), umfassend:
eine Formungseinheit (190), die konfiguriert ist, um eine mehrschichtige Bahn (30) eines absorbierenden Hygieneartikels (10) zu bilden, die eine Rückseitenbahn (114), eine Oberseitenbahn (112) und eine Vielzahl von absorbierenden Kernen (18) umfasst, die zwischen der Oberseitenbahn (112) und der Rückseitenbahn (114) angeordnet sind;
einen Zuführpfad (130), der stromaufwärts von der Formungseinheit (190) entlang einer Vorschubrichtung der Rückseitenbahn (114) angeordnet und so konfiguriert ist, dass er die Rückseitenbahn (114) der Formungseinheit (190) zuführt;
einen Klebeabschnitt-Applikator (140), der entlang des Zuführpfads (130) angeordnet und so konfiguriert ist, dass er eine Vielzahl von Klebeabschnitten (16, 17) auf die Rückseitenbahn (114) anwendet,
wobei jeder Klebeabschnitt (16, 17) eine abnehmbare Deckschicht (4) und eine auf diese Deckschicht (4) aufgebrachte Klebeschicht (5) umfasst;
erste Andruckelemente (150), die entlang des Zuführpfads (130) angeordnet und so konfiguriert sind, dass sie die Klebstoffbereiche (16, 17) mit einer ersten Kraft, die einen vorbestimmten Wert aufweist, gegen die Rückseitenbahn (114) drücken;
wobei die Formungseinheit (190) zweite Andruckelemente (210) umfasst, die so konfiguriert sind, dass sie die Rückseitenbahn (114) mit einer zweiten Kraft, die einen Wert unterhalb des vorbestimmten Werts aufweist, in Richtung der Oberseitenbahn (112) andrücken.

7. Vorrichtung (100) nach Anspruch 6, wobei:
die Vielzahl von Klebeabschnitten (16, 17) eine Vielzahl von mittleren Klebeabschnitten (16) umfasst; und
der Klebeabschnitt-Applikator (140) so konfiguriert ist, dass er die Vielzahl von mittleren Klebstoffabschnitte (16) in einem mittleren Bereich (114a) der Rückseitenbahn (114) aufbringt, die sich rittlings und entlang einer Mittelachse (H) der Rückseitenbahn (114) erstreckt.

8. Vorrichtung (100) nach Anspruch 6 oder 7, wobei:
die Vielzahl von Klebeabschnitten (16, 17) eine Vielzahl von peripheren Klebeabschnitten (17) umfasst;
der Klebeabschnitt-Applikator (140) so konfiguriert ist, dass er die Vielzahl an peripheren Klebeabschnitten (17) an zwei peripheren Bereichen (114b) der Rückseitenbahn (114) anwendet, die auf gegenüberliegenden Seiten im Hinblick auf einen mittleren Abschnitt (114a) der Rückseitenbahn (114) angeordnet sind, der sich über und entlang einer Mittelachse (H) der Rückseitenbahn erstreckt. (114) wobei die Vorrichtung (100) ferner Folgendes umfasst:
einen Auslasspfad (230), der stromabwärts von der Formungseinheit (190) entlang der Vorschubrichtung angeordnet und so konfiguriert ist, dass er die mehrschichtige Bahn (30) von der Formungseinheit (190) aufnimmt;
ein Schneidelement (240), das entlang des Auslasspfads (230) angeordnet und konfiguriert ist zum Schneiden der beiden peripheren Bereiche (114b) umfasst, um eine Vielzahl von Flügeln (13, 15) von absorbierenden Hygieneartikeln (10) zu definieren, die jeweilige periphere Klebeabschnitte (17) der Vielzahl von peripheren Klebeabschnitten (17) umfassen.

9. Vorrichtung (100) nach Anspruch 8, wobei die Vielzahl von Flügeln (13, 15) eine Vielzahl von gegenüberliegender Flügelpaare (13, 15) umfasst, wobei die Vorrichtung (100) ferner Folgendes umfasst:
ein Faltelement (250), das entlang des Auslasspfads (230) platziert und so konfiguriert ist, dass es die Flügel (13, 15) jedes Flügelpaars (13, 15) aufeinander zu faltet;
einen Klebepflaster-Applikator (270), der entlang des Auslasspfads (230) platziert ist, und konfiguriert, um einen Klebepflaster (19) auf die peripheren Klebeabschnitte (17) der gefalteten Flügel (13, 15) jedes Flügelpaares (13, 15) anzuwenden, um die gefalteten Flügel (13, 15) miteinander zu verbinden.

10. Vorrichtung (100) nach einem der Ansprüche 6 bis 9, umfassend einen Applikator für eine Polyvinylalkoholschicht (170), der entlang des Zuführpfads (130) zwischen dem Klebeabschnitt-Applikator (140) und der Formungseinheit (190) platziert und so konfiguriert ist, dass er eine Schicht aus Polyvinylalkohol (14a) auf die Rückseitenbahn (114) auf der den Klebeabschnitten (16, 17) gegenüberliegenden Seite anwendet.

## Revendications

1. Procédé de fabrication d'un article hygiénique absorbant (10), comprenant :
l'application d'une pluralité de parties adhésives (16, 17) sur une bande de feuille de support (114), chaque partie adhésive (16, 17) comprenant une feuille de recouvrement amovible (4) et une couche de colle (5) superposée à ladite feuille de recouvrement (4) ;
puis, la formation d'une bande multicouche (30) d'article hygiénique absorbant comprenant ladite bande de feuille de support (114), une bande de feuille supérieure (112), une pluralité de noyaux absorbants (18) disposés entre ladite bande de feuille supérieure (112) et ladite bande de feuille de support (114), et ladite pluralité de parties adhésives (16, 17) appliquées à ladite bande de feuille de support (114),
lesdites parties adhésives (16, 17) étant disposées sur ladite bande de feuille de support (114) sur un côté opposé par rapport à ladite pluralité de noyaux absorbants (18) ;
avant de former ladite bande multicouche (30), la pression desdites parties adhésives (16, 17) contre ladite bande de feuille de support (114) avec une première force ayant une valeur prédéterminée ; la formation de ladite bande multicouche (30) comprenant la pression de ladite bande de feuille de support (114) contre ladite bande de feuille supérieure (112) avec une seconde force ayant une valeur inférieure à ladite valeur prédéterminée.

2. Procédé selon la revendication 1,
ladite bande de feuille de support (114) comprenant une zone centrale (114a) s'étendant à cheval et le long d'un axe central (H) de ladite bande de feuille de support (114) ;
l'application de ladite pluralité de parties adhésives (16, 17) comprenant l'application d'une pluralité de parties adhésives centrales (16) au niveau de ladite zone centrale (114a).

3. Procédé selon l'une quelconque des revendications précédentes,
ladite bande de feuille de support (114) comprenant une zone centrale (114a) s'étendant à cheval et le long d'un axe central (H) de ladite bande de feuille de support (114) et deux zones périphériques (114b) disposées sur des côtés opposés par rapport à ladite zone centrale (114a) ;
l'application de ladite pluralité de parties adhésives (16, 17) comprenant l'application d'une pluralité de parties adhésives périphériques (17) au niveau desdites deux zones périphériques (114b) ;
ledit procédé comprenant en outre, après la formation de ladite bande multicouche (30), la découpe desdites deux zones périphériques (114b) pour définir une pluralité d'ailes (13, 15) d'articles hygiéniques absorbants (10) comprenant des parties adhésives périphériques (17) respectives de ladite pluralité de parties adhésives périphériques (17).

4. Procédé selon la revendication 3, ladite pluralité d'ailes (13, 15) comprenant une pluralité de paires d'ailes opposées (13, 15), ledit procédé comprenant :
le pliage des ailes (13, 15) de chaque paire d'ailes (13, 15) l'une vers l'autre ;
l'application d'un patch adhésif (19) sur les parties adhésives périphériques (17) des ailes pliées (13, 15) de chaque paire d'ailes (13, 15) afin d'assembler lesdites ailes pliées (13, 15).

5. Procédé selon l'une quelconque des revendications précédentes, comprenant, après l'application de ladite pluralité de parties adhésives (16, 17) et avant la formation de ladite bande multicouche (30), l'application d'une couche d'alcool polyvinylique (14a) sur ladite bande de feuille de support (114) du côté opposé à ladite pluralité de parties adhésives (16, 17).

6. Appareil (100) de fabrication d'un article hygiénique absorbant (10), comprenant :
une unité de formation (190) configurée pour former une bande multicouche (30) d'article hygiénique absorbant (10) comprenant une bande de feuille de support (114), une bande de feuille supérieure (112) et une pluralité de noyaux absorbants (18) disposés entre ladite bande de feuille supérieure (112) et ladite bande de feuille de support (114) ;
un trajet d'alimentation (130) disposé en amont de ladite unité de formation (190) le long d'une direction d'avancement de ladite bande de feuille de support (114) et
configuré pour alimenter ladite bande de feuille de support (114) vers ladite unité de formation (190) ;
un applicateur (140) de parties adhésives disposé le long dudit trajet d'alimentation (130) et configuré pour appliquer une pluralité de parties adhésives (16, 17) sur ladite bande de feuille de support (114),
chaque partie adhésive (16, 17) comprenant une feuille de recouvrement amovible (4) et une couche de colle (5) superposée à ladite feuille de recouvrement (4) ;
des premiers organes de pressage (150) disposés le long dudit trajet d'alimentation (130) et configurés pour presser lesdites parties adhésives (16, 17) contre ladite bande de feuille de support (114) avec une première force ayant une valeur prédéterminée ;
ladite unité de formation (190) comprenant des seconds organes de pression (210) configurés pour presser ladite bande de feuille de support (114) vers ladite bande de feuille supérieure (112) avec une seconde force ayant une valeur inférieure à ladite valeur prédéterminée.

7. Appareil (100) selon la revendication 6,
ladite pluralité de parties adhésives (16, 17) comprenant une pluralité de parties adhésives centrales (16) ; et
ledit applicateur (140) de parties adhésives étant configuré pour appliquer ladite pluralité de parties adhésives centrales (16) au niveau d'une zone centrale (114a) de ladite bande de feuille de support (114) s'étendant à cheval et le long d'un axe central (H) de ladite bande de feuille de support (114).

8. Appareil (100) selon la revendication 6 ou 7,
ladite pluralité de parties adhésives (16, 17) comprenant une pluralité de parties adhésives périphériques (17) ;
ledit applicateur (140) de parties adhésives étant configuré pour appliquer ladite pluralité de parties adhésives périphériques (17) au niveau de deux zones périphériques (114b) de ladite bande de feuille de support (114) disposées sur des côtés opposés par rapport à une zone centrale (114a) de ladite bande de feuille de support (114) s'étendant à cheval et le long d'un axe central (H) de ladite bande de feuille de support (114) ;
ledit appareil (100) comprenant en outre :
une voie de sortie (230) disposée en aval de ladite unité de formation (190) le long de ladite direction d'avancement et configurée pour recevoir ladite bande multicouche (30) de ladite unité de formation (190) ;
un organe de coupe (240) disposé le long de ladite voie de sortie (230) et configuré pour couper lesdites zones périphériques (114b) pour définir une pluralité d'ailes (13, 15) d'articles hygiéniques absorbants (10) comprenant des parties adhésives périphériques (17) respectives de ladite pluralité de parties adhésives périphériques (17).

9. Appareil (100) selon la revendication 8, ladite pluralité d'ailes (13, 15) comprenant une pluralité de paires d'ailes opposées (13, 15), ledit appareil (100) comprenant en outre :
un organe de pliage (250) placé le long de ladite voie de sortie (230) et configuré pour plier les ailes (13, 15) de chaque paire d'ailes (13, 15) l'une vers l'autre ;
un applicateur de patchs adhésifs (270) placé le long de ladite voie de sortie (230) et configuré pour appliquer un patch adhésif (19) sur les parties adhésives périphériques (17) des ailes pliées (13, 15) de chaque paire d'ailes (13, 15) afin d'assembler lesdites ailes pliées (13, 15) l'une à l'autre.

10. Appareil (100) selon l'une quelconque des revendications 6 à 9, comprenant un applicateur (170) de couche d'alcool polyvinylique placé le long dudit trajet d'alimentation (130) entre ledit applicateur (140) de parties adhésives et ladite unité de formation (190) et configuré pour appliquer une couche d'alcool polyvinylique (14a) sur ladite bande de feuille de support (114) du côté opposé auxdites parties adhésives (16, 17).
